# EUROPEAN PATENT SPECIFICATION

(11) **EP 3 391 861 B1**
(45) Date of publication and mention of the grant of the patent: **01.01.2020**
(21) Application number: 16874957.0
(22) Date of filing: 14.12.2016
(51) Int. Cl.: A61F 5/449

(54) **DEVICE FOR SECURING IN AN OSTOMY FOR THE PREVENTION OF HERNIAS**
VORRICHTUNG ZUR BEFESTIGUNG IN EINEM STOMA ZUR VORBEUGUNG VON HERNIEN
DISPOSITIF DE FIXATION À UTILISER AVEC UNE STOMIE POUR LA PRÉVENTION D'HERNIES

(30) Priority: 17.12.2015 ES 201531826
(43) Date of publication of application: 24.10.2018
(73) Proprietor: Fundación para la Investigación Biomedica del Hospital 12 de Octubre, 28041 Madrid (ES)
(72) Inventor: ALMENARA PULIDO, Juan, 28350 Madrid (ES)
(74) Representative: Carvajal y Urquijo, Isabel
(86) International application number: PCT/ES2016/070888
(87) International publication number: WO 2017/103309

(56) References cited:
- CN-U- 202 605 092
- GB-A- 2 495 736
- US-A1- 2012 136 324
- US-A1- 2014 148 771
- US-A1- 2015 065 971
- US-B1- 7 166 091

## Description

### Field of the invention

The present invention relates to a device as described in the claims intended to prevent the appearance of hernias in ostomized patients which is easy to apply on patients who have or may have a parastomal hernia.

### Background of the invention

A parastomal hernia is an incisional hernia associated with the stoma. Different factors are involved in its development such as errors in the surgical technique, postoperative complications or individual characteristics of the patient. It may develop in any stoma, although it is more frequent in colostomies and terminal stomas.

It is present in more than 50% of cases and only 10 to 15% of the same are surgically remedied. The rest of the cases are addressed with conservative measures: flexible collector devices and compression garments and control of the peristomal area.

Devices intended for compression and securing of hernias in ostomized patients are already known.

US 2015/065971 discloses a device for ostomy applications comprising a floating ring, a flange and a belt. The floating ring rests in coplanar fashion against the surface of the flange and contains means for attachment such as grommets for attachment on a tensioning belt to fasten the ostomy appliance to the body. With respect to the flange and the belt, said device provides support to the anatomy surrounding the stoma, and is therefore suitable for securing in an ostomy for the prevention of hernias, especially for the prevention of parastomal hernias.

Document GB20080005056 "Ostomy pouch with handle" describes a handle fixed to the front part of the pouch which facilitates the placement of the wafer on the stoma, followed by the compression of the stoma and hernia during simple pressing with the palm of the hand. The device is more intended for containing effluent and not for the prevention and control of a parastomal hernia.

Document AU2013354536 "An adaptable ostomy base plate", in turn, makes known an adaptable ostomy base plate comprising a flexible top film, at least one elastic adhesive disposed on the top film and at least one release liner, the base plate having at least a first convex section that can be inverted to a concave section to be used for especially demanding skin surface conditions, such as in ostomists suffering from hernia. This device does not effectively cover the need present for the patient who has or may have a parastomal hernia since it only protects the area of the stoma.

Document CN201790930 "multifunctional ostomy bag" describes a multifunctional ostomy bag capable of avoiding complications such as abdominal ostomy hernia, incisional hernia and the like. Similar to the previous devices not solving the problem set out, the device is intended to contain effluent.

Document GB20050017921 "Ostomy support garment" describes a garment which provides physical support for regions e.g. hernia regions, whilst allowing trouble-free access to the stoma. These types of garments are not supported by this type patient as they are uncomfortable, many drawbacks being encountered during its use.

Document FR20090003257 "Abdominal wall's hernia retaining device" describes a device which responds to the need to contain the hernia in patients who suffer from a colostomy. This device, like the previous one, is placed directly over the area of the ostomy, compresses the same and makes it more vulnerable to leaks of effluent.

Document US20150088081 "Stoma Shield for ostomy patients" describes a device for covering a stoma, allowing greater maneuverability of the patient without the need to cut the flow of the ostomy bag, its main function is still to protect, consequently the need presented is not covered by this device, in addition, the securing system to the body of the patient is not suitable and is uncomfortable since it can be easily displaced from the area of the ostomy.

Document US20140276500 "Medical Device Method of Making and Using the Same" describes a device for covering a stoma like the previous device, which has an improved securing system to the body of the patient and although it is configured to prevent or minimize the hernia and leaks, its main function is to be a protection system of the ostomy area.

In none of the cited cases is the appearance and development of hernias effectively prevented in ostomized patients.

### Description of the invention

The present invention has the object of providing a device designed to solve the problem posed, preventing the appearance and development of a parastomal hernia in ostomized patients.

The device of the invention is described in the claims and comprises two elements: an ostomy base intended to be supported on the abdominal wall at both sides of the stoma, housing the ostomy bag and a securing strap with an anatomical shape and suitable dimensions, easy to use for the user whose strap is intended to serve as securing means of the ostomy base to the body of a patient.

The ostomy base is composed of a rigid elongated plate, based on a sanitarily compatible material which is formed longitudinally in the shape of a bridge with a curved central section limited between two approximately flat and coplanar end sections which have a greater thickness than the central section and can protrude through its external surface with respect to the convex surface of the central section.

According to a preferred embodiment, the end sections of the ostomy base are equipped, on their internal surface, with an elastically compressible coating to serve as soft supports on the body of the patient.

According to another characteristic of the invention, the curved central section has through-holes, preferably arranged in parallel alignments and close to one of the curved edges of said central section.

The end sections of the ostomy base will preferably be equipped on their external surface with an antislip and preferably adhesive coating, for example Velcro.

In terms of the securing strap, it will be flexible and elastic and will be equipped at its ends with mutual securing means, for example based on adhesive coatings such as Velcro. This strap can be longitudinally curved to facilitate its adaptation around the body of a patient.

### Brief description of the drawings

In order to complement the description being made and with the object of helping to better understand the characteristics of the invention, in accordance with a preferred practical embodiment thereof, said description is accompanied, as an integral part thereof, by a set of figures where, in an illustrative and nonlimiting manner, the following has been represented:
Figure 1 is an external perspective view of an ostomy base according to the invention.
Figure 2 is an interior perspective view of the same base.
Figure 3 is an exploded view of the device of Figure 1, with the elements of which it is composed.
Figure 4 shows the positioning of the ostomy base in perspective with respect to the securing strap.
Figure 5 is a profile view of the securing strap and ostomy base.
Figure 6 shows a front elevation of the ostomy base.

### Detailed description of an embodiment

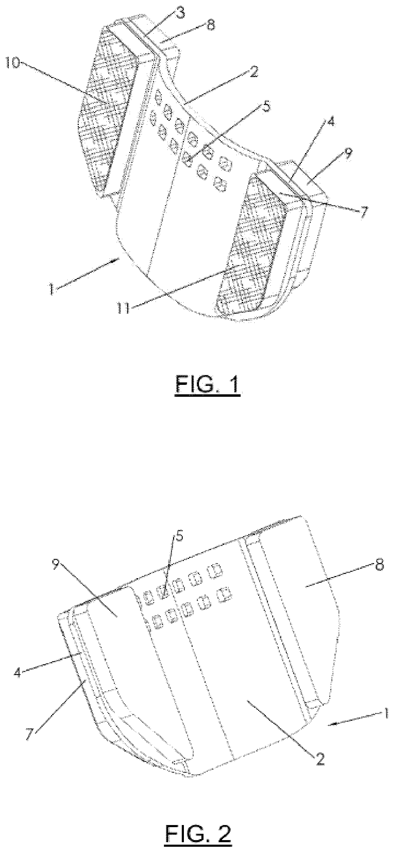

The ostomy base (1) is shown in Figures 1 to 3 which forms part of the invention. This base is composed of a rigid elongated plate which is formed longitudinally in the shape of a bridge, by a curved central section (2), limited between approximately flat and coplanar end sections (3 and 4).

The central section has holes (5) which are distributed in parallel alignments and close to one of the edges of said section, coinciding with the position occupied by the gas outlet valve and drain filter of an ostomy bag.

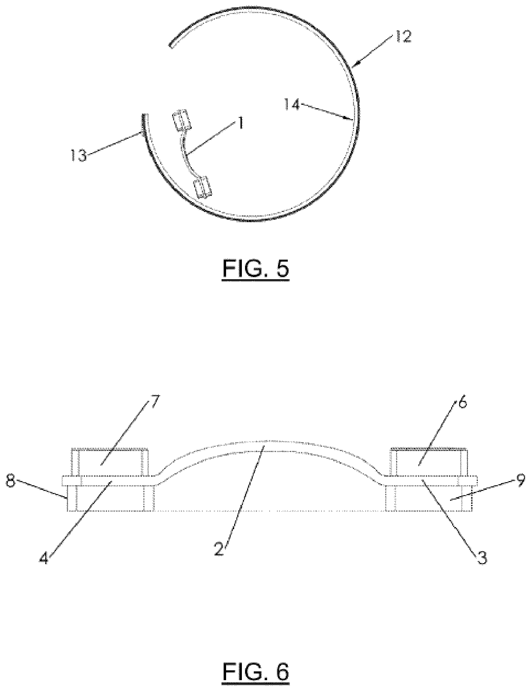

The end sections (3 and 4) have a greater thickness than the central section (2) and form portions (6 and 7) externally which can protrude externally with respect to the convex surface of the central section (2), as is represented in Figure 5. On its interior surface, these end sections (3 and 4) have two internal coatings (8 and 9) applied in the manner of pads based on an elastically compressible material which is soft to touch, for example a biocompatible silicon sponge which can be fixed over the surfaces of the end sections (3 and 4) by means of an adhesive.

The plate forming the base (1) can be manufactured by means of injection molding, for example by means of a thermoplastic for sanitary use which does not have excessive retraction.

The portions (6 and 7) are equipped with an antislip and preferably adhesive external coating (10-11), for example Velcro.

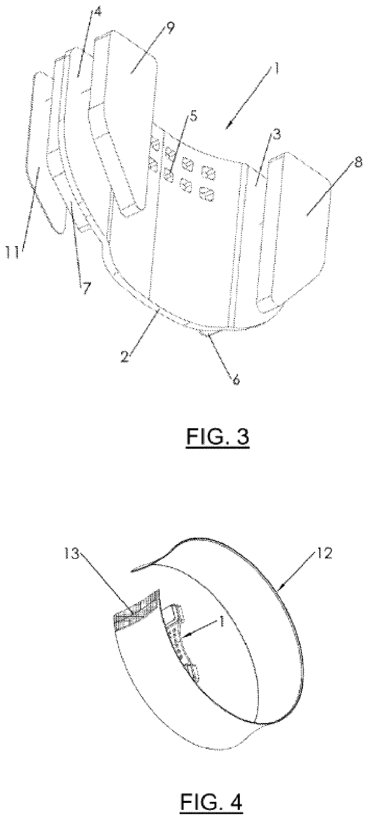

In Figures 4 and 5, the flexible and elastic securing strap (12) is shown, for example based on a neoprene textile and preferably curved section for its easy adaptation to the perimeter of the body of the user, on the ostomy base (1). This strap has securing means at its ends, for example based on an external piece (13) of Velcro and a compatible interior layer (14) which will also serve to absorb perspiration and provide comfortable contact with the skin of the user.

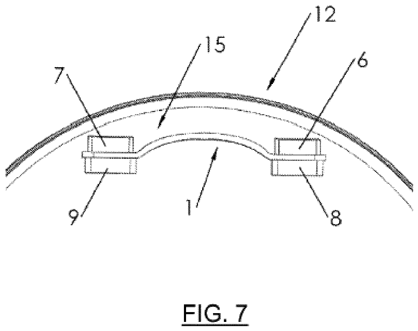

With the described construction, when the securing strap (12) is arranged over the ostomy base (1), Figure 7, between both components there is a space (15) sufficient for allowing the outlet of gases through the holes (5) of said base, preventing the concentration of gases on the peristomal area.

The external coating (10-11) of the portions (3 and 4) enable good securing of the ostomy base to be achieved by means of the securing strap (12) owing to the interior layer (14) of said strap.

The device of the invention enables an effective joining to be achieved between the element forming it. Therefore, the particular design of the ostomy base is going to allow the ostomy area to be covered whilst it is surrounded with the ostomy strap providing uniform pressure without excessive compression occurring at both sides of the same, achieving, on the one hand, better tolerance for the patient of the device, and most importantly, preventing the appearance and development of a parastomal hernia in these types of patients.

Another advantages of the device of the invention is that the user carrying the stoma could place the device on themselves easily, the ostomy base is supported on the securing strap of the base, over the side where the stoma is located, the stoma being adhered to the strap itself in a stable manner by means of Velcro. Once fixed, the entire strap will surround the waist just as easily, the assembly being adjusted over the waist of the user.

## Claims

1. A device for securing in an ostomy for the prevention of hernias comprising an ostomy base (1) and a securing strap (12) of said base, **characterized in that** the ostomy base is composed of an elongated rigid plate which has the shape of a bridge, with a curved central section (2), equipped with through-holes (5) and two approximately flat and coplanar end sections (3-4) which have a greater thickness than the central section (1); and whose securing strap (12) is elastic and flexible and has mutual securing means at its ends.

2. The device according to claim 1, **characterized in that** the end sections (3-4) of the ostomy base (1) have secured at their internal surface two internal coatings (8-9) based on biocompatible silicon sponge.

3. The device according to claim 1, **characterized in that** the holes (5) of the curved central section (2) of the ostomy base (1) are distributed in parallel rows and close to one of the curved edges of said central section.

4. The device according to claim 1, **characterized in that** the end sections (3-4) of the ostomy base have on their external surface an adhesive coating (10-11).

5. The device according to claim 1, **characterized in that** the securing strap (12) has on its ends, as securing means, opposing adhesive coatings.

6. The device according to claim 1, **characterized in that** the end sections (3-4) of the ostomy base (1) form externally portions (6-7) which protrude with respect to the convex surface of the central section (2).

## Patentansprüche

1. Vorrichtung zur Befestigung in einem Stoma zur Vorbeugung von Hernien, umfassend eine Stomabasis (1) und einen Befestigungsriemen (12) der genannten Basis, **dadurch gekennzeichnet, dass** die Stomabasis aus einer länglichen starren Platte in Brückenform und einem gekrümmten mittleren Abschnitt (2) besteht, der mit Durchgangslöchern (5) und zwei annähernd flachen und koplanaren Endabschnitten (3, 4) ausgestattet ist, die eine größere Dicke als der mittlere Abschnitt (1) aufweisen; wobei der Befestigungsriemen (12) elastisch und flexibel ist und an seinen Enden beidseitig Befestigungsmittel aufweist.

2. Vorrichtung nach Anspruch 1, **dadurch gekennzeichnet, dass** an der Innenfläche der Endabschnitte (3, 4) der Stomabasis (1) zwei Innenbeschichtungen (8, 9) basierend auf biokompatiblem Silikonschwamm aufgebracht sind.

3. Vorrichtung nach Anspruch 1, **dadurch gekennzeichnet, dass** die Löcher (5) des gekrümmten mittleren Abschnitts (2) der Stomabasis (1) in parallelen Reihen in der Nähe einer der gekrümmten Kanten des genannten mittleren Abschnitts angeordnet sind.

4. Vorrichtung nach Anspruch 1, **dadurch gekennzeichnet, dass** die Endabschnitte (3, 4) der Stomabasis an ihrer Außenfläche eine Haftbeschichtung (10, 11) aufweisen.

5. Vorrichtung nach Anspruch 1, **dadurch gekennzeichnet, dass** der Befestigungsriemen (12) an seinen Enden gegenüberliegende Haftbeschichtungen als Befestigungsmittel aufweist.

6. Vorrichtung nach Anspruch 1, **dadurch gekennzeichnet, dass** die Endabschnitte (3, 4) der Stomabasis (1) außenseitig Bereiche (6, 7) bilden, die im Vergleich zur konvexen Oberfläche des mittleren Abschnitts (2) vorstehen.

## Revendications

1. Dispositif de fixation à utiliser avec une stomie pour la prévention d'hernies comprenant une base de stomie (1) et une sangle de fixation (12) de ladite base,
**caractérisé en ce que** la base de stomie est composée d'une plaque rigide allongée qui a la forme d'un pont, avec une section centrale courbe (2), équipée de trous traversants (5) et deux sections d'extrémité (3-4) approximativement plates et coplanaires qui ont une épaisseur supérieure à la section centrale (1), et dont la sangle de fixation (12) est élastique et souple et présente à ses extrémités des moyens de fixation mutuelle.

2. Dispositif selon la revendication 1, **caractérisé en ce que** les sections d'extrémité (3-4) de la base de stomie (1) ont fixé à leur surface interne deux revêtements internes (8-9) à base d'éponge de silicone biocompatible.

3. Dispositif selon la revendication 1, **caractérisé en ce que** les trous (5) de la section centrale courbe (2) de la base de stomie (1) sont répartis en rangées parallèles et près de l'un des bords courbes de ladite section centrale.

4. Dispositif selon la revendication 1, **caractérisé en ce que** les sections d'extrémité (3-4) de la base de stomie présentent sur leur surface externe un revêtement adhésif (10-11).

5. Dispositif selon la revendication 1, **caractérisé en ce que** la sangle de fixation (12) présente à ses extrémités, en tant que moyens de fixation, des revêtements adhésifs opposés.

6. Dispositif selon la revendication 1, **caractérisé en ce que** les sections d'extrémité (3-4) de la base de stomie (1) forment extérieurement des parties (6-7) qui dépassent par rapport à la surface convexe de la section centrale (2).
